# EUROPÄISCHE PATENTANMELDUNG

(11) **EP 1 857 070 A1**
(43) Veröffentlichungstag der Anmeldung: **21.11.2007**
(21) Anmeldenummer: 06010292.8
(22) Anmeldetag: 18.05.2006
(51) Int. Cl.: A61B 19/00

(54) **Kontaktfreie medizintechnische Registrierung mit Distanzmessung**

(71) Anmelder: BrainLAB AG, 85551 Kirchheim/Heimstetten (DE)
(72) Erfinder: Woerlein, Swen, 80637 München (DE)
(74) Vertreter: Rögner, Jürgen

(57) **Zusammenfassung**

Die Erfindung betrifft eine medizintechnische Registrierungsvorrichtung mit einer Ortsbestimmungseinrichtung (1, 7, 8; 9), mittels welcher die räumliche Position von Behandlungsgeräten, behandlungsunterstützenden Geräten oder Patienten bzw. Patiententeilen erfasst werden kann, und mit einer Datenverarbeitungseinheit (4), welche erfasste Positionen von Patienten bzw. Patiententeilen entsprechenden Punkten eines akquirierten Patienten-Bilddatensatzes zuordnet, wobei die Registrierungsvorrichtung eine Abstandsmessvorrichtung (6) aufweist, deren räumliche Position mittels der Ortsbestimmungsvorrichtung (1, 7, 8) erfasst wird und welche die Abstandsdaten für gemessene Punkte an die Datenverarbeitungseinheit (4) übergibt. Sie betrifft ferner ein Verfahren zur medizintechnischen Registrierung von Patienten bzw. Patiententeilen zu entsprechenden Punkten eines akquirierten Patienten-Bilddatensatzes, bei dem mit einer Ortsbestimmungseinrichtung (1, 7, 8; 9) die räumliche Position einer Abstandsmessvorrichtung (6) erfasst wird, und bei dem die Abstandsdaten für gemessene Punkte an eine Datenverarbeitungseinheit (4) übergeben werden, die aus der räumlichen Position der Abstandsmessvorrichtung (6) und den Abstandsdaten die räumliche Lage der Punkte ermittelt.

## Beschreibung

Die Erfindung betrifft die kontaktfreie medizintechnische Registrierung. Insbesondere betrifft sie eine medizintechnische Registrierungsvorrichtung sowie ein Verfahren zur medizintechnischen Registrierung von Patienten bzw. Patiententeilen.

Um Ärzten bei ihrer Behandlung eine visuelle Unterstützung bereitstellen zu können, werden Bilddaten verwendet, die mit so genannten bildgebenden medizintechnischen Verfahren ermittelt werden. Beispiele für solche bildgebenden Verfahren sind Computertomographie- oder Kernspintomographieverfahren, aber auch Röntgenverfahren, PET- und SPECT-Verfahren gehören dazu. Mit diesem Verfahren erhält man einen Bilddatensatz des Patienten, in den meisten Fällen einen dreidimensionalen Bilddatensatz mit darauf abgebildeten inneren und äußeren Strukturen. Um diesen Datensatz während der Behandlung verwenden zu können, das heißt um Behandlungsgeräte oder behandlungsunterstützende Geräte im richtigen Positionsverhältnissen zu diesem Bilddatensatz anzeigen zu können, muss eine so genannte Registrierung vor Ort, d.h. bei der Behandlung bzw. davor erfolgen. Bei einer solchen Registrierung werden die Strukturen aus dem Bilddatensatz und entsprechende Punkte am oder im Patienten einander zugeordnet und in einem speziellen Raumkoordinatensystem festgelegt. Nun können Instrumente oder andere Behandlungseinrichtungen visuell in korrekter Lagezuordnung zu aquirierten Bilddaten angezeigt und damit dem behandelnden Arzt eine Bildunterstützung zur Verfügung gestellt werden.

Um eine solche Registrierung durchführen zu können, kann man grundsätzlich mit einem schon registrierten Zeigeinstrument bestimmte Punkte, die auch Landmarken genannt werden, an einem Patienten anfahren (zum Beispiel mit der Spitze eines Pointers) und dem unterstützenden Computersystems, also meist einem medizinisch-technischen Navigationssystem, dann mitteilen, welcher Punkt dem realiter angefahrenen Punkt im Bilddatensatz entspricht. Nach dem Anfahren und Zuordnen mehrer Punkte kann dann eine dreidimensionale Registrierung vorgenommen werden. Eine solche herkömmliche Registrierungsmethode ist aber aufwändig und zeitraubend. Sie bedingt auch ein optisches oder magnetisches Tracking (Positionsverfolgung) im Navigationssystem.

Aus der US 6,033,415 ist ein Registrierungsverfahren bekannt, das der Durchführung robotischer Orthopädieprozeduren dienen soll. Dabei wird mit einem Zeigerwerkzeug (Digitalisierungseinrichtung), das an einem robotischen Gelenkarm angeordnet ist, ein Punkt nach dem anderen auf einem Knochen angefahren, und der mit Gelenksensoren ausgestattete Roboter erfasst dabei die äußere Form des Knochens in seinem Koordinatensystem. Diese äußere Form kann dann dazu verwendet werden, ein vorab akquiriertes Abbild des Knochens auf die erfasste tatsächliche Lage zu transformieren. Es wird ferner vorgeschlagen, mittels eines fest an einem Roboter angebrachten, kontaktfreien Digitalisierungsapparates (Ultraschall- oder Lasersystem) digitalisierte Knochendatensätze zu erstellen. Einzelheiten oder praktische Umsetzungen hierfür sind nicht angegeben.

Ein großer Nachteil der in diesem Dokument beschriebenen Kontaktregistrierung mit der Spitze eines Roboterarms ist aber wiederum der relativ große Aufwand beim Anfahren aller notwendigen Punkte. Die dargestellte Ausführungsform für das kontaktfreie Registrieren bzw. Digitalisieren ist schon deshalb problematisch, weil der fest am Roboter angeordnete Digitalisierungsapparat immer nur einen Teil des Patienten, und diesen Teil nur aus einer einzigen Perspektive sehen kann. Praktikabel ist eine solche kontaktfreie Registrierungsmethode nicht, weil mit ihr aus dem vorher genannten Grund verschiedenste Körperteile nicht erfasst werden können.

Eine weitere kontaktfreie Registrierungsmethode ist der EP 1 142 536 B1 bekannt, wobei ein Patient in einem medizintechnischen Navigationssystem mittels aufgestrahlter Lichtpunkte registriert wird. Weil die Lichtpunkte auf der Oberfläche des Patienten selbst als Registrierungspunkte dienen, müssen sie für das verwendete Tracking-System so gut wie möglich sichtbar sein, was sich bei manchen Anwendungen als schwierig herausstellt.

Es ist eine Aufgabe der vorliegenden Erfindung, eine kontaktfreie medizinische Registrierung bereitzustellen, die genau und umfassend arbeitet und ohne zu großen Aufwand durchführbar ist. Diese Aufgabe wird erfindungsgemäß durch eine medizintechnische Registrierungsvorrichtung gemäß dem Anspruch 1 sowie ein Registrierungsverfahren gemäß dem Anspruch 9 gelöst. Die Unteransprüche definieren bevorzugt Ausführungsformen der Erfindung.

Eine medizintechnische Registrierungsvorrichtung nach der vorliegenden Erfindung weist eine Ortsbestimmungseinrichtung auf, mittels welcher die räumliche Position von Behandlungsgeräten, behandlungsunterstützenden Geräten oder Patienten bzw. Patiententeilen erfasst werden kann. Solche Ortsbestimmungseinrichtungen sind alle Einrichtungen, die feststellen können, wo im Raum sich der Patient oder ein Behandlungsgerät bzw. ein behandlungsunterstützendes Gerät befindet, zum Beispiel ein Instrument oder seine Spitze. Ferner umfasst die Registrierungsvorrichtung eine Datenverarbeitungseinheit, welche erfasste Positionen von Patienten bzw. Patiententeilen entsprechenden Punkten eines aquirierten Patienten-Bildsatzes zuordnet. Erfindungsgemäß weist die Registrierungsvorrichtung eine Abstandsmessvorrichtung auf, deren räumliche Position mittels der Ortsbestimmungsvorrichtung erfasst wird und welche die Abstandsdaten für gemessene Punkte an die Datenverarbeitungseinheit übergibt.

Mit anderen Worten ist es bei der erfindungsgemäßen Registrierung die Abstandsmessung, die es gestattet, Punkte zu erfassen, ohne diese direkt mit einem Instrument anfahren zu müssen. Solche Abstandsmessvorrichtungen sind schon auf dem Markt erhältlich oder können leicht an die Bedürfnisse der erfindungsgemäßen Ausgestaltung angepasst werden. Weil mittels der Ortsbestimmungsvorrichtung die räumliche Position der Abstandmessvorrichtung erfasst werden kann, kann diese Vorrichtung bewegbar, bzw. handhabbar, also in einer nicht fixierten Ausführungsform bereitgestellt werden. Hierdurch wird es möglich, auch Punkte oder Flächen zu registrieren, die für ein optisches Tracking-System nicht sichtbar sind, zum Beispiel Punkte oder Flächen innerhalb eines Resektionsbereiches oder andere optisch "hinterschnittene" Flächen.

Bei einer Ausführungsform der Erfindung ist die Ortsbestimmungseinrichtung ein medizinisches Trackingsystem, insbesondere ein optisches Trackingsystem und speziell ein Kamara-Trackinsystem, bei dem mittels einer stereoskopischen-Kameraeinheit Referenzanordnungen an Behandlungsgeräten, behandlungsunterstützenden Geräten oder Patienten bzw. Patiententeilen positionell bestimmt werden. Anzumerken ist hier, dass durch dieses Trackingsystem nicht die Punkte zur Registrierung selbst aufgenommen werden, sondern lediglich die Abstandsmessvorrichtung erfasst wird, wobei kaum Probleme mit der Sichtbarkeit auftreten. An der Abstandsmessvorrichtung kann hierzu beispielsweise eine Referenzanordnung angeordnet sein.

Gemäß einer weiteren Ausführungsform der Erfindung wird die Abstandsvorrichtung an einem Gelenkarm angeordnet, wobei die Ortsbestimmungseinrichtung Positionsbestimmungssensoren oder Positionsänderungssensoren umfasst, insbesondere Winkelerfassungssensoren, die in den Gelenken des Gelenkarms angeordnet sind. Der Gelenkarm kann der Roboterarm eines medizinischen Roboters sein. Diese Ortsbestimmung mit Hilfe von Gelenksensoren kann allein stehend eingesetzt werden, es kann aber auch eine Ortsbestimmungseinrichtung eingesetzt werden, die sowohl einen Arm mit Gelenksensoren als auch ein optisches Tracking der Abstandsmessvorrichtung umfasst. Letztere Ausgestaltung ermöglicht eine redundante gegenseitige Ergänzung.

Die anfangs angesprochene Datenverarbeitungseinheit kann ein Teil eines medizinischen Navigationssystems sein, das bei solchen Anwendungen meist ohnehin zur Verfügung steht.

Die Abstandsmessvorrichtung kann verschiedene Ausgestaltungen annehmen. Sie kann einerseits ein Laserstrahl-Distanzmesser, insbesondere ein Laserstrahl-Distanzmesser zur Messung eines linearen Abstandes sein. Solche Geräte sind verfügbar und einfach aufgebaut und lassen sich leicht in ein erfindungsgemäßes . System integrieren. Andererseits ist es für die erfindungsgemäße Abstandsmessvorrichtung grundsätzlich nur notwendig, dass sie Daten über den Abstand eines Punktes bereitstellt, und auch andere Geräte können eine solche Funktion liefern. Deshalb kann jedes Abstandsbestimmungssystem verwendet werden, insbesondere auch ein Fokus-Abstandssystem eines räumlich ortsbestimmbaren, medizintechnischen Mikroskops. Beispielsweise kann hierzu eine Autofokus-Einrichtung eines solchen Mikroskops herangezogen werden, die auf einen bestimmten Punkt fokussiert. Dieser Autofokus-Punkt bzw. der Autofokus-Abstand ist im Mikroskopsystem bekannt und kann als Abstand der Registrierung gemäß der vorliegenden Erfindung verwendet werden. Wenn ein Mikroskop verwendet wird, welches Sensoren aufweist, die seine Lage bzw. die Lage seiner funktionellen Teile im Raumkoordinatensystem bestimmbar machen, kann die Abstandsmessung mittels der Fokussierung auch ohne ein äußeres Trackingsystem erfolgen.

Gemäß einem weiteren Aspekt betrifft die vorliegende Erfindung ein Verfahren zur medizintechnischen Registrierung von Patienten bzw. Patiententeilen zu entsprechenden Punkten eines akquirierten Patienten-Bilddatensatzes. Dabei wird mit einer Ortsbestimmungseinrichtung die räumliche Position einer Abstandsmessvorrichtung erfasst und die Abstandsdaten für gemessene Punkte werden an eine Datenverarbeitungseinheit übergeben, die aus der räumlichen Position der Abstandsmessvorrichtung und den Abstandsdaten die räumliche Lage der Punkte ermittelt. Die durch das erfindungsgemäße Verfahren erzielbaren Vorteile entsprechen denen, die oben anhand der entsprechenden Vorrichtung erläutert wurden.

Die Registrierung kann durch eine (separate) oder die oben schon genannte Datenverarbeitungseinheit durchgeführt werden, wobei eine Punktzuordnung zwischen den akquirierten Bilddatensatz und mehreren Punkten und deren räumliche Lage ermittelt wurde, mithilfe eines Surface-Matching-Verfahrens erfolgt. Solche Surface-Matching-Verfahren oder -Programme sind bekannt und verfügbar und sie können anhand von speziell geformten Oberflächen eine genaue Zuordnung der Strukturen aus der Abstandserfassung und aus dem akquirierten Bilddatensatz verwirklichen. Allgemein ist eine solche Registrierung natürlich für vorab akquirierte Bilddatensätze möglich, aber auch für während der Behandlung zusätzlich akquirierte Bilddatensätze.

Die bei einer erfindungsgemäßen Ausführungsvariante des Verfahrens verwendete Ortsbestimmungseinrichtung kann ein medizintechnisches Trackingsystem sein, wie es weiter oben schon beschrieben wurde, und in gleicher Weise kann die räumliche Position der Abstandsmessvorrichtung erfahrungsgemäß durch eine an ihr angeordnete Referenzeinrichtung erfasst werden. Es ergibt sich ferner verfahrensmäßig die Möglichkeit, mit der Abstandsmessvorrichtung als einen Registrierungs-Ausgangspunkt einen Punkt zu erfassen, der einer Referenzanordnung zugeordnet ist, die am Patienten angebracht ist oder in einem bekannten Positionsverhältnis zum Patienten steht. Die anfängliche Punktzuordnung bei der Registrierung wird hierdurch erleichtert. Es ist anzumerken, dass natürlich auch mit der Abstandsmessvorrichtung eine so genannte Punkt-zu-Punkt-Registrierung durchgeführt werden kann, indem für besondere Landmarken der Abstand erfasst und die erfindungsgemäße Registrierung durchgeführt wird.

Die räumliche Lageerfassung für die Punkte kann gemäß einer Ausführungsform mittels einer Abstandsmessvorrichtung erfolgen, die an einem Roboterarm eines medizinischen Gelenkarm-Roboters angeordnet ist, der Positionsbestimmungssensoren oder Positionsänderungssensoren, insbesondere Winkelerfassungssensoren in den Gelenken des Gelenkarms aufweist. Ferner besteht die Möglichkeit, die Lageerfassung automatisch oder halbautomatisch durch Ansteuerung des Gelenkarm-Roboters durchzuführen, wobei innerhalb eines vorgegebenen Zielbereichs aufeinander folgend die Lage einer ausreichenden Anzahl von Punkten ermittelt wird, mittels der eine Registrierung erfolgen kann.

Die Erfindung gestattet es also, einen Patienten auf vorab oder intra-operativ akquirierte Bilddatensätze zu registrieren, indem ein kalibriertes kontaktfreies Abstandsmessgerät verwendet wird, zum Beispiel ein so genannter Laser-Range-Finder. Das Abstandsmessgerät kann den Abstand zu einem Punkt an der Patientenkörperoberfläche messen, während die relative Position des Abstandsmessgerätes bezüglich des Patienten bekannt ist (zum Beispiel durch ein Trackingsystem, das die Position des Abstandsmessgerätes und des Patienten verfolgt). Die Information der schon akquirierten Punkte (zum Beispiel Ausbreitung, Differenziation, etc.) kann verwendet werden, um festzustellen, ob schon eine ausreichende Punktezahl für eine erfolgreiche Registrierung akquiriert worden ist.

Durch das Wiederholen dieses Vorgangs können mehrere Punkte mit bekannten relativen Positionen gegenüber dem Patienten erfasst werden, und diese Punkte können dann als ein Teil der Eingabe eines Surface-Matching-Algorithmus genutzt werden, dessen anderen Eingabeteil der Patienten-Datensatz ist. Der Algorithmus führt dann ein Matching durch, das heißt eine Anpassung der Punkte an die Daten des Datensatzes, und man erhält schließlich das Positionsverhältnis des Patienten zum Bilddatensatz.

Um eine automatische oder halbautomatische Registrierung bereitstellen zu können, kann das Abstandsmessgerät an einem robotischen Arm montiert werden. Der robotische Arm kann dann in einer solchen Weise positioniert werden, dass das Abstandsmessgerät sich auf einen interessierenden Bereich der Oberfläche richtet, die registriert werden soll. Dieser Bereich, der im Moment von Interesse ist, kann auch automatisch bestimmt werden, beispielsweise durch die Verwendung einer Lokalisierungsvorrichtung (Referenzanordnung), die an dem Patienten befestigt ist und als Start- oder Ausgangsposition verwendet wird. Hierauf kann der Roboterarm sich dann in Bezug auf die vordefinierte Position bewegen, beispielsweise in einem Radius um die vordefinierte Position, und das Abstandsmessgerät wird ausgelöst, um Registrierungspunkte zu erfassen. Der Vorgang kann für mehrere interessierende Bereiche wiederholt werden. Die Information der schon akquirierten Registrierungspunkte kann wiederum verwendet werden, um zu bestimmen, ob eine ausreichende Anzahl von Punkten für eine erfolgreiche Registrierung akquiriert worden sind und der Akquirierungsprozess kann auch automatisch beendet werden, um ein Surface-Matching-Verfahren zu starten.

Wenn der Roboterarm sein eigenes Koordinatensystem hat, wobei die räumliche Beziehung unterschiedlicher Positionen bekannt ist, und der Patient in einer fixierten (aber nicht unbedingt bekannten) Position zum Roboterkoordinatensystem liegt, ist ein "äußeres Tracking" nicht unbedingt nötig. Das "innere Tracking", das durch die bekannten Gelenkpositionen des Roboters bereitgestellt wird, genügt, und unter Verwendung der Informationen des Abstandsmessgerätes können die Systeme die Positionen der akquirierten Punkte im Roboterkoordinatensystem bestimmen. Wenn dann das Positionsverhältnis des Patienten zum Bilddatensatz durch ein Surface-Matching bestimmt worden ist, ist ebenfalls die Positionsrelation zum Roboterkoordinationsystem bekannt ist und es kann navigiert werden.

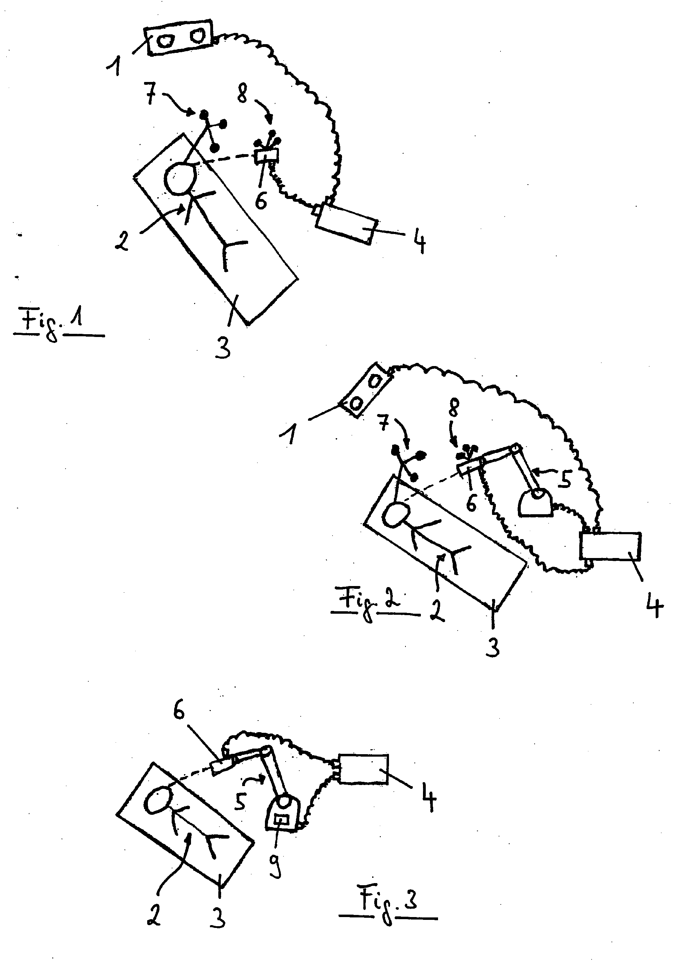

Die Erfindung wird im Weiteren anhand verschiedener Ausführungsformen und unter Bezugnahme auf die beiliegenden Zeichnungen näher erläutert. Sie kann alle hierin beschriebenen Merkmale einzeln oder in jedweder Kombination umfassen. In den Zeichnungen zeigen:
- Figur 1: eine schematische Ansicht eines erfindungsgemäßen Registrierungssystems in einer Ausführungsform, bei der die Abstandsmessvorrichtung frei geführt werden kann;
- Figur 2: eine erfindungsgemäße Ausgestaltung mit einer von außen getrackten Abstandsmessvorrichtung an einem Roboterarm;
- Figur 3: eine Ausführungsform der Erfindung mit einem Roboterarm ohne äußeres Trackingsystem.
Die schematische Darstellung der Figur 1 zeigt ein erfindungsgemäßes Registrierungssystem in einer ersten Ausgestaltung. Ein Patient 2 liegt auf einer Liege 3, und an dem Patienten ist im zu registrierenden Bereich eine Referenzanordnung 7 angeordnet, die zu einem optischen Trackingsystem gehört. Das optische Trackingsystem ("äußeres Trackingsystem") ist ebenso schematisch dargestellt und mit dem Bezugszeichen 1 versehen. Es weist eine stereoskopische Kameraeinheit und eine Logik auf, die beobachteten Punkten, wie zum Beispiel den Referenzkugeln auf der Referenzanordnung 7 räumliche Positionen zuordnen kann. Ferner ist in Figur 1 eine Abstandsmessvorrichtung 6 dargestellt, die im vorliegenden Beispiel ein Laser-Abstandsmesser sein kann. Die gestrichelte Linie deutet an, dass der Abstand zwischen dem Abstandsmesser 6 und einem Punkt am Kopf des Patienten gemessen wird. Auch der Laser-Abstandsmesser 6 ist mit einem Referenzstern (Referenzanordnung) 8 versehen und somit kann die Position des Abstandsmessers 6 im Raum durch das Trackingsystem 1 festgestellt werden. Sowohl das Trackingsystem 1 als auch der Laser-Abstandsmesser 6 sind mit einer Datenverarbeitungseinheit 4, verbunden, die separat dargestellt ist, aber auch die Datenverarbeitungseinheit eines hier nicht gezeigten medizintechnischen Navigationssystems sein kann.

Der Abstandsmesser 6 aus Figur 1 ist frei beweglich angeordnet, er kann also beispielsweise mit der Hand geführt werden, um einen bestimmten Punkt zu registrieren. Dies gestattet die Registrierung auch schwer sichtbarer Punkte am Patienten, beispielsweise innerhalb eines schon vorgenommenen Einschnittes. Weil die Position des Abstandsmessers 6 über die Referenzanordnung 8 im System bekannt ist, und der lineare Abstand (gestrichelte Linie) zu jedem Punkt gemessen und an die Datenverarbeitungseinheit 4 weitergemeldet werden kann, kann auch die aktuelle Lage des Punktes selbst, dessen Abstand gerade gemessen wird, im Raumkoordinatensystem festgestellt werden, und wenn eine ausreichende Anzahl von Punkten in dieser Weise erfasst wurde, lässt sich mittels eines Surface-Matching-Verfahrens die so abgebildete Struktur einer entsprechenden Struktur eines Bilddatensatzes zuordnen, der vorab mittels eines bildgebenden Verfahrens akquiriert wurde. Damit wird eine kontaktfreie und sehr unaufwändig gestaltbare Registrierung auch für schwer zugängliche Punkte möglich.

Eine andere Ausführungsform eines Systems mit einer erfindungsgemäßen Registrierungsvorrichtung ist in Figur 2 gezeigt, wobei in den Figuren gleiche Bezugszeichen auf gleiche Vorrichtungen deuten. Bei diesem System ist der Abstandsmesser 6 am Armende eines Gelenkarm-Roboters 5 befestigt. Die Verwendung des Roboters 5 gestattet nun eine automatische bzw. halbautomatische Akquirierung von Patienten-Registrierungspunkten. So kann beispielsweise ausgehend von einem-Punkt, der durch die Referenzanordnung 7 bestimmt wird, der Roboterarm automatisch oder nach einer Aktivierung mit Hilfe des Abstandsmessers 6 eine Anzahl von Punkten in einer Region von Interesse aufnehmen, indem er automatisch mehrere Punkte in dieser Region abfährt. Der durch die Referenzanordnung 7 bestimmte, vorher genannte Punkt definiert dabei einen vorbestimmten Ausgangspunkt. Wenn genügend Punkte am Patienten akquiriert worden sind, um eine Zuordnung zu entsprechenden Bilddaten im Bilddatensatz zu gestatten, kann der Roboter automatisch mit der Akquirierung von Patienten-Registrierungspunkten aufhören oder den selben Vorgang an einer anderen ebenso vordefinierten, interessierenden Region fortsetzen.

Ein anderer Aspekt der Verwendung des Roboters ist derjenige, der sich auf sein eigenes, inneres Koordinatensystem bezieht. Es stehen medizintechnische Roboter zur Verfügung, die ein Raumkoordinatensystem haben und welche ausgehend von einem Nullpunkt die Bewegungen ihrer Armabschnitte oder der daran angebrachten funktionellen Einrichtungen über Gelenksensoren in den Gelenken zwischen den Armen bzw. zwischen dem ersten Armteil und der Roboterbasis verfolgen können. Ein solcher Roboter weiß also in seinem eigenen Koordinatensystem ("inneres Trackingsystem") selbst, wo die Abstandsmessvorrichtung 6 liegt und über den dort gemessenen Abstand auch wo .der derzeit akquirierte Patientenpunkt liegt. Wenn, wie in der Ausführungsform nach Figur 2, zusätzlich noch ein "äußeres Tracking" über das Trackingsystem 1 und die Referenzanordnung 8 am Abstandsmesser 6 erfolgt, können sich diese beiden Ortsbestimmungssysteme redundant ergänzen, oder es kann beim Ausfall eines Systems auf die Daten des anderen System zurückgegriffen werden. Dazu muss Anfangs lediglich ein Abgleich der Koordinatensysteme des Roboters 5 und des Trackingsystems 1 erfolgen.

Weil, wie oben schon erläutert, Roboter eine eigene Ortbestimmungsvorrichtung mit Gelenksensoren aufweisen können, sind auch Ausführungsformen denkbar, wie sie beispielsweise in Figur 3 dargestellt sind. Diese Ausführungsform benutzt kein äußeres Trackingsystem mehr sondern lediglich das Gelenk-Koordinatensystem des Roboters, das mittels einer Datenverarbeitungseinheit 9 Positionsdaten für den Abstandsmesser 6 und mit Hilfe des von ihm gemeldetem Abstandes auf Positionsdaten über den gerade vermessenen Punkt am Patienten verarbeiteten und an die Datenverarbeitungseinheit 4 ausgebend kann.

Es ist, wie in Figur 3 auch dargestellt, ebenfalls möglich, dass die Datenverarbeitungseinheiten und der Abstandsmesser 6 direkt verbunden werden. Auch in diesem Fall erhält die Datenverarbeitungseinheit 4 alle Daten, die notwendig sind, um angefahrene bzw. vermessene Punkte auf entsprechende Strukturen in einem Bilddatensatz zu registrieren.

Bei einem solchen Verfahren muss die Lage des Patienten nicht unbedingt im System bekannt sein. Wenn der Patient gut fixiert ist, können in der vorher beschriebenen Weise Patientenpunkte akquiriert und eine Registrierung durchgeführt werden, und dann könnte die Abstandsmessvorrichtung 6 am Roboter 5 durch ein medizinisches Instrument ersetzt werden, mit dem navigiert und bildunterstützt gearbeitet werden kann.

## Patentansprüche

1. Medizintechnische Registrierungsvorrichtung mit einer Ortsbestimmungseinrichtung (1, 7, 8; 9), mittels welcher die räumliche Position von Behandlungsgeräten, behandlungsunterstützenden Geräten oder Patienten bzw. Patiententeilen erfasst werden kann, und mit einer Datenverarbeitungseinheit (4), welche erfasste Positionen von Patienten bzw. Patiententeilen entsprechenden Punkten eines akquirierten Patienten-Bilddatensatzes zuordnet, **dadurch gekennzeichnet, dass** die Registrierungsvorrichtung eine Abstandsmessvorrichtung (6) aufweist, deren räumliche Position mittels der Ortsbestimmungsvorrichtung (1, 7, 8) erfasst wird und welche die Abstandsdaten für gemessene Punkte an die Datenverarbeitungseinheit (4) übergibt.

2. Registrierungsvorrichtung nach Anspruch 1, **dadurch gekennzeichnet, dass** die Ortsbestimmungseinrichtung ein medizintechnisches Trackingsystem, insbesondere ein optisches Trackingsystem (1, 7, 8) aufweist, speziell ein Kamera-Trackingsystem, bei dem mittels einer stereoskopischen Tracking-Kameraeinheit (1) Referenzanordnungen (7, 8) an Behandlungsgeräten, behandlungsunterstützenden Geräten oder Patienten bzw. Patiententeilen positionell bestimmt werden.

3. Registrierungsvorrichtung nach Anspruch 2, **dadurch gekennzeichnet, dass** an der Abstandsmessvorrichtung (6) eine Referenzanordnung (8) angeordnet ist.

4. Registrierungsvorrichtung nach einem der Ansprüche 1 bis 3, **dadurch gekennzeichnet, dass** die Abstandsmessvorrichtung (6) an einem Gelenkarm angeordnet ist, wobei die Ortsbestimmungseinrichtung Positionsbestimmungssensoren oder Positionsänderungssensoren, insbesondere Winkelerfassungssensoren in den Gelenken des Gelenkarms aufweist.

5. Registrierungsvorrichtung nach Anspruch 4, **dadurch gekennzeichnet, dass** der Gelenkarm ein Roboterarm (5) eines medizintechnischen Roboters ist.

6. Registrierungsvorrichtung nach einem der Ansprüche 1 bis 5, **dadurch gekennzeichnet, dass** die Datenverarbeitungseinheit ein Teil eines medizintechnischen Navigationssystems ist.

7. Registrierungsvorrichtung nach einem der Ansprüche 1 bis 6, **dadurch gekennzeichnet, dass** die Abstandsmessvorrichtung (6) ein Laserstrahl-Distanzmesser, insbesondere ein Laserstrahl-Distanzmesser zur Messung eines linearen Abstandes ist.

8. Registrierungsvorrichtung nach einem der Ansprüche 1 bis 3 oder 6, **dadurch gekennzeichnet, dass** die Abstandsmessvorrichtung ein Abstandsbestimmungssystem, insbesondere ein Fokus-Abstandsbestimmungssystem eines räumlich ortsbestimmbaren, medizintechnischen Mikroskops ist.

9. Verfahren zur medizintechnischen Registrierung von Patienten bzw. Patiententeilen zu entsprechenden Punkten eines akquirierten Patienten-Bilddatensatzes, bei dem mit einer Ortsbestimmungseinrichtung (1, 7, 8; 9) die räumliche Position einer Abstandsmessvorrichtung (6) erfasst wird, und bei dem die Abstandsdaten für gemessene Punkte an eine Datenverarbeitungseinheit (4) übergeben werden, die aus der räumlichen Position der Abstandsmessvorrichtung (6) und den Abstandsdaten die räumliche Lage der Punkte ermittelt.

10. Verfahren nach Anspruch 9, bei dem die Registrierung durch eine oder die genannte Datenverarbeitungseinheit (6) durchgeführt wird, wobei eine Punktzuordnung zwischen dem akquirierten Bilddatensatz und mehreren Punkten, deren räumliche Lage ermittelt wurde, mit Hilfe eines Surface-Matching-Verfahrens erfolgt.

11. Verfahren nach Anspruch 10, bei dem die Ortsbestimmungseinrichtung durch ein medizintechnisches Trackingsystem, insbesondere ein optisches Trackingsystem (1, 7, 8) erfolgt, speziell ein Kamera-Trackingsystem, durch welches mittels einer stereoskopischen Tracking-Kameraeinheit (1) Referenzanordnungen (7, 8) an Behandlungsgeräten, behandlungsunterstützenden Geräten oder Patienten bzw. Patiententeilen positionell bestimmt werden.

12. Verfahren nach Anspruch 11, bei dem die räumliche Position der Abstandsmessvorrichtung (6) durch eine an ihr angeordnete Referenzanordnung (8) erfasst wird.

13. Verfahren nach Anspruch 11 oder 12, bei dem mit der Abstandsmessvorrichtung (6) als ein Registrierungs-Ausgangspunkt ein Punkt erfasst wird, der einer Referenzanordnung zugeordnet ist, die am Patienten angebracht ist oder in einem bekannten Positionsverhältnis zum Patienten steht.

14. Verfahren nach einem der Ansprüche 9 bis 11, bei dem die räumliche Lageerfassung für die Punkte mittels einer Abstandsmessvorrichtung (6) erfolgt, die an einem Roboterarm (5) eines medizintechnischen Gelenkarm-Roboters (5) angeordnet ist, der Positionsbestimmungssensoren oder Positionsänderungssensoren, insbesondere Winkelerfassungssensoren in den Gelenken des Gelenkarms aufweist.

15. Verfahren nach Anspruch 14, bei dem die Lageerfassung automatisch oder halbautomatisch durch Ansteuerung des Gelenkarm-Roboters (5) erfolgt, wobei innerhalb eines vorgegebenen Zielbereichs aufeinander folgend die Lage einer ausreichenden Anzahl von Punkten ermittelt wird, mittels der eine Registrierung erfolgen kann.
